# EUROPEAN PATENT APPLICATION

(11) **EP 1 284 142 A1**
(43) Date of publication of application: **19.02.2003**
(21) Application number: 01119444.6
(22) Date of filing: 13.08.2001
(51) Int. Cl.: A61K 35/78, A61P 31/00

(54) **Pharmaceutical composition for treatment of infective immunologic disorders**

(71) Applicant: Viking Agricultural Products Company, Njombe (TZ)
(72) Inventor: Kadowela, Rongius Pangasta, Njombe (TZ)
(74) Representative: Nielsen, Leif L.

(57) **Abstract**

This invention provides compositions of plant extracts to be used for prophylaxis and treatment of infective immunologic disorders including HIV, AIDS and AIDS related complex in addition to boils and pimples. Furthermore, methods for production of these compositions are disclosed including pharmaceutical preparations and medicaments.

## Description

### FIELD OF THIS INVENTION

The present invention relates to the use of plant compositions for the prophylaxis and treatment of patients suffering from infective immunologic disorders. In particular the present invention relates to the use of plant compositions for the prophylaxis and treatment of one or more of the disorders Human Immunodeficiency Virus (HIV) disease and Acquired Immune Deficiency Syndrome (AIDS) and AIDS related complex in addition to boils and pimples. The present invention also embraces pharmaceutical compositions comprising these plant compositions and methods of producing and using said compositions.

### BACKGROUND OF THIS INVENTION

One object of the present invention is to furnish compositions of compounds that can effectively be used in the treatment and prophylaxis of patients suffering from infective immunologic disorders. In particular this invention furnish compositions for prophylaxis and treatment of HIV, AIDS and AIDS related complex.
Other important examples of immunologic disorders treatable by means of this invention comprise boils (abscesses) and pimples (acne).

HIV is an abbreviation for Human Immunodeficiency Virus. AIDS is a pandemic immunosuppressive disease, which results in life threatening opportunistic infections and malignancies. HIV has in clearly been demonstrated to be the etiologic agent of AIDS. According to "Report on the global HIV/AIDS epidemic by the Joint Nations Program on HIV/AIDS at the XIIIth International AIDS Conference, Durban, South Africa, July 2000" it is estimated that the total number worldwide of HIV infected patients is about 35 millions. In the same report it is estimated that 80% of these patients live in Africa and that they are geographically concentrated within an area South of Sahara.

As to the development of the AIDS disease, the virus during the course of the disease kills the so-called helper cells (CD4+ T cells). The helper cells are also central against many other infectious diseases in the immunologic defence of the human body. During the development of the disease there will be an increased loss of the helper cells in the blood. Said loss is progressive and leads eventually to a stage in which the body is rendered incapable of resisting many bacterial, fungal, viral and parasitical invasions implying that patients suffering from HIV, AIDS or AIDS related complex are in an imminent risk of acquiring opportunistic infections. As per medical definition any HIV-infected individual with an CD4+ T cell count less than 200/µL is diagnosed as being infected with AIDS, regardless of the presence of any obvious symptoms or opportunistic diseases.

Furthermore the viral infection facilitates certain cancer diseases, such as e.g. invasive cervical cancer, Burkitt's Lymphoma and Kaposi's sarcoma. A retrovirus, designated human immunodeficiency virus (HIV-2) also being the causative agent of AIDS has recently been isolated. HIV-1 type m, type n, or type o and HIV-2 are presently the best-known etiologic agents of AIDS.

Several agents have been reported to inhibit the growth of the human immunodeficiency virus. One category hereof is inhibitors of the nucleoside analogue reverse transcriptase, abbreviated NRTIs (Nucleoside analogue Reverse Transcriptase Inhibitors. Examples hereof comprise e.g. zidovudine, didanosine, zalcitabine, stavudine, lamivudine, abacavir. A second category consists of drugs called none nucleoside reverse transcriptase inhibitors and examples hereof comprise nevirapine, delavirdine, and efavirenz. A further category comprises protease inhibitors, e.g. saquinavir, ritonavir, indinavir and nelfinavir. As a common and recognized practice today (according to Harrison Medical Textbook, McGraw-Hill, 2000), combinations of 3 drugs from 2 of the above mentioned 3 categories are used, for example one none nucleoside reverse transcriptase inhibitor combined with 2 nucleoside analogue reverse transcriptase inhibitors (NRTIs) or one protease inhibitor in combination with 2 nucleoside analogue reverse transcriptase inhibitors (NRTIs).

However, a considerable drawback by the above-mentioned agents is that they are relatively costly. Furthermore, they have been shown to have toxic effects in the range from life-threatening conditions to mild unwanted adverse effects after prolonged use.

Thirdly, with the presently known drugs, which are economic affordable only in the Western part of the world, a very high compliance is mandatory in order to be able to reduce the risk of viral resistance to occur. In reality, experience has disclosed great difficulties in keeping these patients in compliance with a given medical administration regimen. Thus, these compounds have shown to be - although effective when ingested properly - less suitable in treating the patients infected with HIV, AIDS or AIDS related complex as the number of the so-called dropouts is considerable high.

On the other hand, in poor socio-economic countries access to an effective treatment with presently known drugs is low as the costs of prolonged treatment are far above the threshold of a normal health budget in this part of the world.

In summary, the currently known drug regimens available for treatment of HIV, AIDS or AIDS related complex are complicated and expensive and potentially toxic to the patients. As the majority of the persons infected with HIV, AIDS or AIDS related complex lives in poor socio-economic areas of the world, there is an urgent need for the access to a less toxic, less expensive and more effective anti-HIV medication.

The artisan of the present invention has surprisingly found a composition and a method for preparing said composition and a regimen for administration hereof, which hitherto has revealed no toxic effects, will be less expensive to prepare and more effective in comparison with presently known treatments. Moreover, the regimen adhered to in accordance with the present invention is easily administrated.

In the present invention AIDS related complex comprises the finding in a patient of a number of CD4+ T cells of less than 500 per µl of blood, which normally is accompanied of significant but often minor infections, in addition to diseases such as e.g. Peripheral Neuropathy, Cytomegalovirus (CMV) and Candidias of bronchi, trachea or lungs

Herbal and other vegetable preparations are presently used and have for centuries been used for treatment of various diseases in Africa. In continuation hereof, the search for and interest in a natural treatment to human health problems has resulted in research of herbal and other natural sources as to their active substances, the toxicity and the pharmacological activities.

One important object of the present invention is to provide a plant composition for the prophylaxis and/or treatment of HIV, AIDS or AIDS related complex in addition to boils and pimples and/or a method for the prophylaxis and/or treatment of patients suffering hereof by the administration of said plant composition.

In this context the treatment and/or prophylaxis of HIV, AIDS and/or AIDS related complex shall mean treatment and/or prophylaxis of a clinically visible and verifiable disease as well as at a sub-clinical stage, which comprises e.g. an imminent state, i.e. a state that does not show any manifest clinical signs of the disease, but is clinically assessed as a threat or imminence.

A second object of this invention is addressed to the use of said plant composition for the prophylaxis and/or treatment of AIDS and HIV in addition to boils and pimples by the administration of said plant composition.

A third object of this invention is to provide a method for the prophylaxis and/or treatment of AIDS and AIDS related complex in addition to boils and pimples to comprising the step of administering to an animal or a human being a pharmaceutically acceptable and therapeutically effective amount of said plant preparation.

A fourth object of this invention is to provide a pharmaceutical composition for the prophylaxis and/or treatment of HIV, AIDS and AIDS related complex in addition to boils and pimples comprising a pharmaceutically acceptable and a therapeutically effective amount of said plant preparation.

A fifth object of this invention is to provide a medicament for the prophylaxis and/or treatment of HIV, AIDS and AIDS related complex in addition to boils and pimples comprising a pharmaceutically acceptable and a therapeutically effective amount of said plant preparation.

A sixth object of this invention is to provide a method for an in vitro inhibition of HIV infected human T lymphocytes and mononuclear phagocytic lineage cells infected with HIV.

### BRIEF DESCRIPTION OF THIS INVENTION

It has, surprisingly, been found that compositions further characterised below can be used as a remedy against infective immunologic disorders comprising HIV, AIDS or AIDS related complex. Said compositions are to be administered to a human being or an animal in need of a treatment in an amount that is therapeutically effective. Boils and pimples are other examples of immunologic disorders treatable by means of this invention.

The present invention is based in part on the discovery that a preparation comprising an extract containing at least one or more than one active substance of one or more of the following plants: Rauvolfia caffra Sond., Rhamnus prinoïdes L'Hérit., Toddalia asiatica (L.) Lam. and Zanthoxylum deremense (Engl.) Kokwaro has proved to be effective against immunologic disorders comprising HIV, AIDS and AIDS related complex in addition to boils and pimples.

In one aspect of the invention a composition is uncovered for use in the prophylaxis and treatment of HIV, AIDS and AIDS related complex in addition to boils and pimples. The composition comprises an extract containing at least one or more than one active substance of one or more of the following plants: Rauvolfia caffra Sond., Rhamnus prinoïdes L'Hérit., Toddalia asiatica (L.) Lam. and Zanthoxylum deremense (Engl.) Kokwaro.

In a second aspect of the invention use of one or more of said compositions - i.e. compositions that comprise an extract containing at least one or more than one active substance of one or more of the following plants: Rauvolfia caffra Sond., Rhamnus prinoïdes L'Hérit., Toddalia asiatica (L.) Lam. and Zanthoxylum deremense (Engl.) Kokwaro.- is/are claimed for the manufacture of a medicament for the treatment of HIV, AIDS and AIDS related complex in addition to boils and pimples.

In a third aspect of the invention a method is provided for treating animal or human beings suffering from HIV, AIDS and AIDS related complex in addition to boils and pimples.

In a fourth aspect of the invention a medicament is provided for the treatment of diseases and conditions of HIV, AIDS and AIDS related complex in addition to boils and pimples.

In a fifth aspect of the invention a pharmaceutical preparation is provided for use in the treatment of animal or human beings suffering from the HIV, AIDS and AIDS related complex in addition to boils and pimples.

In a sixth aspect of the invention a method is provided for in vitro inhibition of HIV infection in human T lymphocyte cells and mononuclear phagocytic lineage cells infected with HIV.

### DETAILED DESCRIPTION OF THIS INVENTION

To facilitate the detailed description of the present invention, it is helpful to set forth definitions of certain terms to be used hereinafter.

AIDS is an abbreviation of Acquired Immune Deficiency Syndrome and in the present context it is to be understood as the condition of any HIV-infected individual with a CD4+ cell counts less than 200/µL. Viral replication continues during the period of clinical latency, and the immunologic function of the HIV-infected individual usually declines progressively. At some point, usually after the CD4+ T cell count has fallen below 500/µL, patients begin to develop the clinical characteristics of the early symptomatic disease, see Table 1 below. Many of these problems can be traced to minor opportunistic infections, not sufficiently indicative of a defect in cell-mediated immunity to be considered AIDS-defining illnesses, while some of them appear to be direct effects of long-standing HIV infection. As used herein, this stage of HIV infection is designated AIDS-related complex.

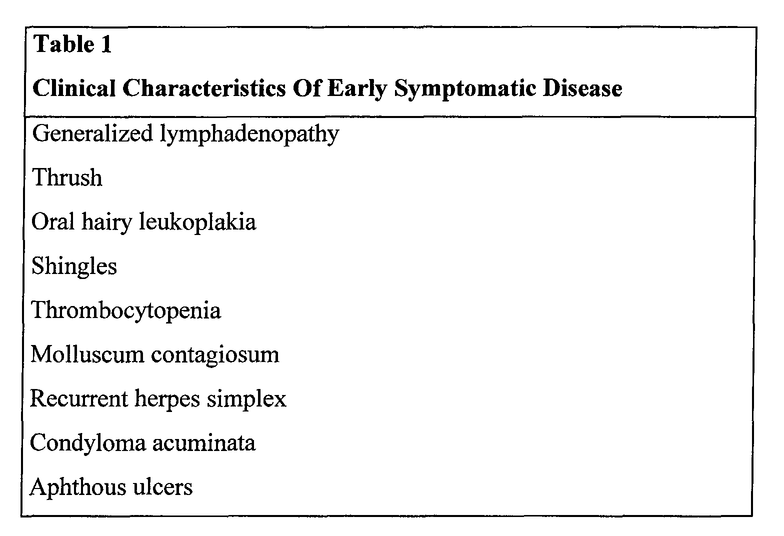

Thus, AIDS related complex is in the present context to be understood as the total selection of diseases, clinical symptoms and conditions with relation to AIDS. HIV is an abbreviation of Human Immunodeficiency Virus and in the present context it is to be understood as the etiologic agent of AIDS.

AIDS was originally defined prior to the identification of HIV by the presence of a reliably diagnosed "opportunistic" disease that is at least moderately predictive of an underlying defect in cell-mediated immunity in the absence of known causes of underlying immune defects, such as iatrogenic immunosuppression or malignant neoplasms. With the identification of HIV as the etiologic agent of AIDS and with the availability of sensitive and specific diagnostic tests for HIV infection, the case definition of AIDS has undergone several revisions. As used herein AIDS is defined on the basis of clinical conditions associated with HIV infection and CD4+ T lymphocyte counts.

The system used to make the diagnosis AIDS is based on three ranges of CD4+ T lymphocyte counts and three clinical categories and is represented by a matrix of nine mutually exclusive categories (see details in Harrison's online, Part 12, Chapter 308, last modified March 12, 2001, McGraw-Hill). Using this system, any HIV-infected individual with a CD4+ T cell count of less than 200/µL has AIDS by definition, regardless of the presence of symptoms or opportunistic diseases (see Table 1).

The clinical categories of HIV Infection (categories A, B and C) comprise the diseases given in Table 2 but are not limited to the quoted diseases.

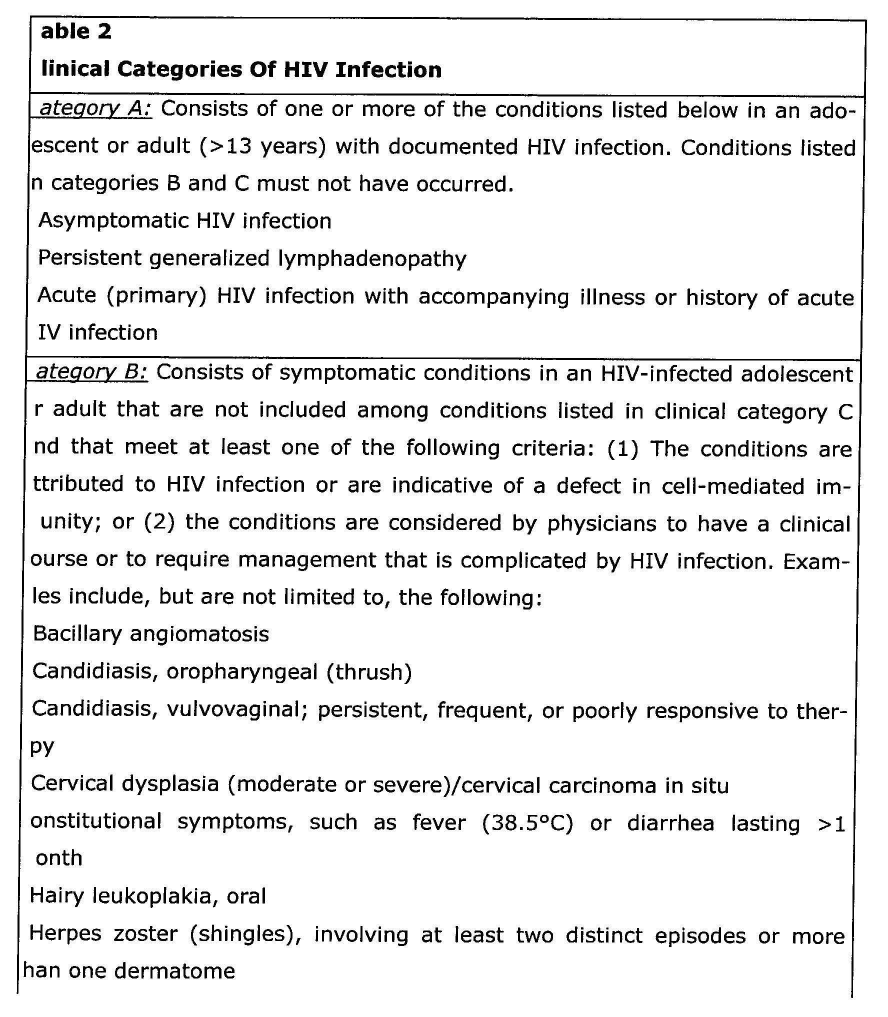

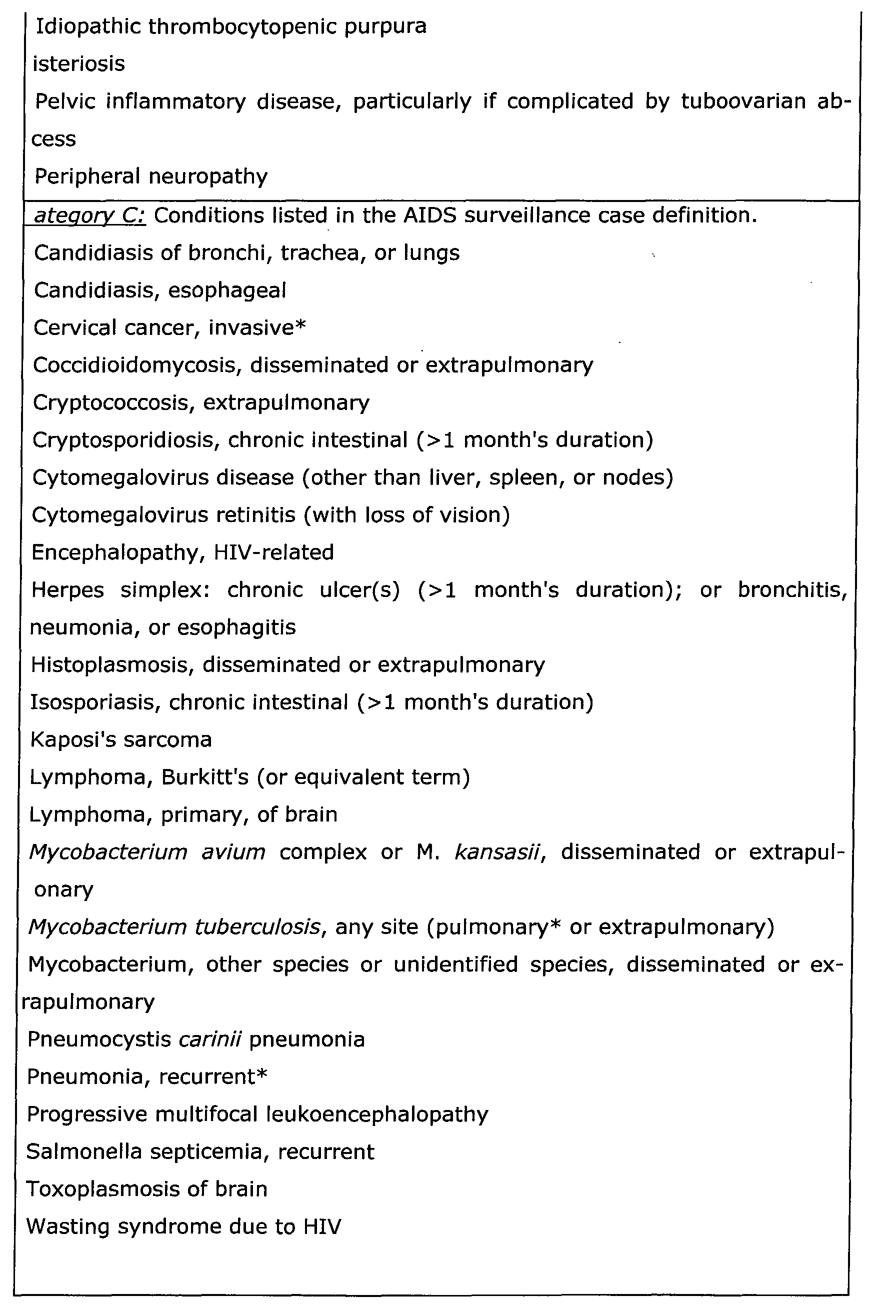

It appears from Table 2, that category C includes as examples pulmonary tuberculosis, recurrent pneumonia, and invasive cervical cancer. Hence, once individuals have had a clinical condition in category B, their disease cannot again be classified as category A, even if the condition resolves; the same holds true for category C in relation to category B.

It appears clearly from the above that these viral infections facilitate certain cancer diseases, such as e.g. invasive cervical cancer, Burkitt's Lymphoma and Kaposi's sarcoma.

In this context the treatment of HIV and/or AIDS and/or AIDS related complex shall mean treatment of a clinically visible and verifiable disease as well as an imminent state, i.e. a state that does not show any manifest clinical signs of the disease, but is clinically assessed as a threat or imminence. Furthermore, treatment as such shall mean actions that imply an improvement of the state. In the case of boils and/or pimples improvement is to be understood as a decrease in number and/or seriousness.

Infective immunologic disorders are to be understood as infective disorders caused by invading micro organism(s) or molecule(s) such as e.g. bacteria, vira, chlamydia, rickettsia, fungi and prions into an previously healthy individual organism. Examples of immunologic disorders comprise HIV, AIDS and AIDS related complex in addition to boils and pimples.

In a first embodiment of the present invention, a composition is provided for the prophylaxis and treatment of infective immunologic disorders such as e.g. HIV, AIDS and AIDS related complex in addition to boils and pimples comprising a therapeutically acceptable composition of a plant material comprising an extract containing at least one or more than one active substance of one or more of the following plants: Rauvolfia caffra Sond., Rhamnus prinoïdes L'Hérit., Toddalia asiatica (L.) Lam. and Zanthoxylum deremense (Engl.) Kokwaro.

In a second embodiment of the invention the use of a composition is claimed for the prophylaxis and treatment of HIV, AIDS and AIDS related complex in addition to boils and pimples comprising a therapeutically acceptable composition of plant material comprising an extract containing at least one or more than one active substance of one or more of the following plants: Rauvolfia caffra Sond., Rhamnus prinoïdes L'Hérit., Toddalia asiatica (L.) Lam. and Zanthoxylum deremense (Engl.) Kokwaro.

In a third embodiment of the invention a method is provided for the prophylaxis and treatment of HIV, AIDS and AIDS related complex in addition to boils and pimples comprising the step of administering to an animal or human being a therapeutically effective amount of a preparation comprising an extract containing at least one or more than one active substance of one or more of the following plants: Rauvolfia caffra Sond., Rhamnus prinoïdes L'Hérit., Toddalia asiatica (L.) Lam. and Zanthoxylum deremense (Engl.) Kokwaro.

In a fourth embodiment of the invention a pharmaceutical composition is claimed for the prophylaxis and treatment of HIV, AIDS and AIDS related complex in addition to boils and pimples comprising a therapeutically acceptable composition of plant material comprising an extract containing at least one or more than one active substance of one or more of the following plants: Rauvolfia caffra Sond., Rhamnus prinoïdes L'Hérit., Toddalia asiatica (L.) Lam. and Zanthoxylum deremense (Engl.) Kokwaro.

In a fifth embodiment of the invention a medicament is claimed for the prophylaxis and treatment of HIV, AIDS and AIDS related complex in addition to boils and pimples comprising a therapeutically acceptable composition of plant material comprising an extract containing at least one or more than one active substance of one or more of the following plants: Rauvolfia caffra Sond., Rhamnus prinoïdes L'Hérit., Toddalia asiatica (L.) Lam. and Zanthoxylum deremense (Engl.) Kokwaro.

In a sixth embodiment of the invention a method is provided for in vitro inhibition of HIV infection in human T lymphocyte cells and mononuclear phagocytic lineage cells infected with HIV. In one assay it has been shown that an extract containing at least one or more than one active substance of one or more of the following plants: Rauvolfia caffra Sond., Rhamnus prinoïdes L'Hérit., Toddalia asiatica (L.) Lam. and Zanthoxylum deremense (Engl.) Kokwaro in a 1:100 dilution significantly is able to inhibit the viral growth of and protect an infected T lymphocyte cell suspension.

The above claimed embodiments 1-6 of the invention will below be described in more detail.

As to the first embodiment of the invention, the composition is made available for the prophylaxis and treatment of infective immunologic disorders comprising diseases such as e.g. HIV, AIDS and AIDS related complex in addition to boils and pimples and comprises a therapeutically acceptable composition of plant material comprising 20-70%, preferably 40-50%, even more preferably 43-47%, of the total volume of the following formulated by volume of Rauvolfia caffra Sond., 20-60%, preferably 35-45%, of the total volume formulated by volume of Rhamnus prinoïdes L'Hérit. up till 30%, preferably 5-15%, of the total volume formulated by volume of Toddalia asiatica (L.) Lam. and up till 30%, preferably 5-15%, of the total volume formulated by volume of Zanthoxylum deremense (Engl.) Kokwaro.

It is to be understood though that the particular volume of each plant may depend upon the potency of each plant.

The potency of each plant is assessed and measured (as follows) by volume of dried plant materiel.

Furthermore, as to the first embodiment of the invention, the composition provided for the prophylaxis and treatment of HIV, AIDS and AIDS related complex in addition to boils and pimples might also according to the present invention as a particular useful composition comprise no or relatively less Zanthoxylum deremense (Engl.) Kokwaro. In this event the content of Zanthoxylum deremense (Engl.) Kokwaro is to be replaced in full or partly by Toddalia asiatica (L.) Lam., which is already a part of the composition. In a preferred embodiment of the invention the amount of Toddalia asiatica (L.) Lam. in said particular useful composition is replaced by half of the amount of Zanthoxylum deremense (Engl.) Kokwaro.

Furthermore, as to the first embodiment of the invention, the pertinent plant material selected for the composition might be dried.

As to the second embodiment of the invention, the use of a composition is provided for the prophylaxis and treatment of HIV, AIDS and AIDS related complex in addition to boils and pimples and it comprises a therapeutically acceptable composition of plant material comprising 20-70%, preferably 40-50%, even more preferably 43-47%, of the total volume of the following formulated by volume of Rauvolfia caffra Sond., 20-60%, preferably 35-45%, of the total volume formulated by volume of Rhamnus prinoïdes L'Hérit. up till 30%, preferably 5-15%, of the total volume formulated by volume of Toddalia asiatica (L.) Lam. and up till 30%, preferably 5-15%, of the total volume formulated by volume of Zanthoxylum deremense (Engl.) Kokwaro.
It is to be understood though that the particular volume of each plant may depend upon the potency of each plant.
The potency of each plant is assessed and measured (as follows) by volume of dried plant materiel.

Furthermore, as to the second embodiment of the invention, the composition provided for the prophylaxis and treatment of HIV, AIDS and AIDS related complex in addition to boils and pimples might also according to the present invention as a particular useful composition comprise no or relatively less Zanthoxylum deremense (Engl.) Kokwaro. In this event the content of Zanthoxylum deremense (Engl.) Kokwaro is to be replaced in full or partly by Toddalia asiatica (L.) Lam., which is already a part of the composition. In a preferred embodiment of the invention the amount of Toddalia asiatica (L.) Lam. in said particular useful composition is replaced by half of the amount of Zanthoxylum deremense (Engl.) Kokwaro.

Furthermore, as to the second embodiment of the invention, the pertinent plant material selected for the composition in use might be dried.

Generally, it applies to the second embodiment of the invention that it also encompasses the use of the pertinent composition for the preparation of a medicament for the prophylaxis and treatment of HIV, AIDS and AIDS related complex in addition to boils and pimples.

As to the third embodiment of the invention, the method provided for the prophylaxis and treatment of HIV, AIDS and AIDS related complex in addition to boils and pimples comprises a therapeutically acceptable composition of plant material comprising 20-70%, preferably 40-50%, even more preferably 43-47%, of the total volume of the following formulated by volume of Rauvolfia caffra Sond., 20-60%, preferably 35-45%, of the total volume formulated by volume of Rhamnus prinoïdes L'Hérit. up till 30%, preferably 5-15%, of the total volume formulated by volume of Toddalia asiatica (L.) Lam. and up till 30%, preferably 5-15%, of the total volume formulated by volume of Zanthoxylum deremense (Engl.) Kokwaro.
It is to be understood though that the particular volume of each plant may depend upon the potency of each plant.
The potency of each plant is assessed and measured (as follows) by volume of dried plant materiel.

Furthermore, as to the third embodiment of the invention, the composition provided for the prophylaxis and treatment of HIV, AIDS and AIDS related complex in addition to boils and pimples might also according to the present invention as a particular useful composition comprise no or relatively less Zanthoxylum deremense (Engl.) Kokwaro. In this event the content of Zanthoxylum deremense (Engl.) Kokwaro is to be replaced in full or partly by Toddalia asiatica (L.) Lam., which is already a part of the composition. In a preferred embodiment of the invention the amount of Toddalia asiatica (L.) Lam. in said particular useful composition is replaced by half of the amount of Zanthoxylum deremense (Engl.) Kokwaro.

Furthermore, as to the third embodiment of the invention, the pertinent plant material selected for the composition in use might be dried.

As to the fourth embodiment of the invention, the pharmaceutical preparation provided for the prophylaxis and treatment of HIV, AIDS and AIDS related complex in addition to boils and pimples comprises a therapeutically acceptable composition of plant material comprises 20-70%, preferably 40-50%, even more preferably 43-47%, of the total volume of the following formulated by volume of Rauvolfia caffra Sond. 20-60%, preferably 35-45%, of the total volume formulated by volume of Rhamnus prinoïdes L'Hérit. up till 30%, preferably 5-15%, of the total volume formulated by volume of Toddalia asiatica (L.) Lam. and up till 30%, preferably 5-15%, of the total volume formulated by volume of Zanthoxylum deremense (Engl.) Kokwaro.
It is to be understood though that the particular volume of each plant may depend upon the potency of each plant.
The potency of each plant is assessed and measured (as follows) by volume of dried plant materiel.

Furthermore, as to the fourth embodiment of the invention, the composition provided for the prophylaxis and treatment of HIV, AIDS and AIDS related complex in addition to boils and pimples might also according to the present invention as a particular useful composition comprise no or relatively less Zanthoxylum deremense (Engl.) Kokwaro. In this event the content of Zanthoxylum deremense (Engl.) Kokwaro is to be replaced in full or partly by Toddalia asiatica (L.) Lam., which is already a part of the composition. In a preferred embodiment of the invention the amount of Toddalia asiatica (L.) Lam. in said particular useful composition is replaced by half of the amount of Zanthoxylum deremense (Engl.) Kokwaro.

Furthermore, as to the fourth embodiment of the invention, the pertinent plant material selected for the composition in use might be dried.

As to the fifth embodiment of the invention, the medicament provided for the prophylaxis and treatment of HIV, AIDS and AIDS related complex in addition to boils and pimples comprises a therapeutically acceptable composition of plant material comprising 20-70%, preferably 40-50%, even more preferably 43-47%, of the total volume of the following formulated by volume of Rauvolfia caffra Sond., 20-60%, preferably 35-45%, of the total volume formulated by volume of Rhamnus prinoïdes L'Hérit. up till 30%, preferably 5-15%, of the total volume formulated by volume of Toddalia asiatica (L.) Lam. and up till 30%, preferably 5-15%, of the total volume formulated by volume of Zanthoxylum deremense (Engl.) Kokwaro.
It is to be understood though that the particular volume of each plant may depend upon the potency of each plant.

The potency of each plant is assessed and measured (as follows) by volume of dried plant materiel.

Furthermore, as to the fifth embodiment of the invention, the composition provided for the prophylaxis and treatment of HIV, AIDS and AIDS related complex in addition to boils and pimples might also according to the present invention as a particular useful composition comprise no or relatively less Zanthoxylum deremense (Engl.) Kokwaro. In this event the content of Zanthoxylum deremense (Engl.) Kokwaro is to be replaced in full or partly by Toddalia asiatica (L.) Lam., which is already a part of the composition. In a preferred embodiment of the invention the amount of Toddalia asiatica (L.) Lam. in said particular useful composition is replaced by half of the amount of Zanthoxylum deremense (Engl.) Kokwaro.

Furthermore, as to the fifth embodiment of the invention, the pertinent plant material selected for the composition in use might be dried.

Such medicaments comprise in addition to said composition pharmaceutically acceptable additives and/or vehicles and they may suitably be formulated for oral, intranasal, pulmonal, intravenous, intramuscular, subcutaneous, rectal or vaginal administration.

The present invention thus relates inter alia to pharmaceutical compositions and medicaments. Strategies in formulation development of medicaments and compositions based on the compounds of the present invention correspond to formulation strategies known to people skilled in the art. Potential problems and the guidance required to overcome these problems are treated in several textbooks, such as e.g. "Therapeutic Peptides and Protein Formulation. Processing and Delivery Systems", Ed. A.K. Banga, Technomic Publishing AG, Basel, 1995.

Categories of pharmaceutical formulations comprise in the present invention a dry formulation such as e.g. powder (to be dissolved when to be ingested), A capsule and a tablet. In addition, the formulation according to the present invention may be liquid, such as e.g. an aqueous solution.

As to the sixth embodiment of the invention a method is provided for in vitro inhibition of HIV infection in human T lymphocyte cells and mononuclear phagocytic lineage cells infected with HIV. By using this method the pertinent cells infected with or possible infected with HIV are to be treated with at least one or more than one active substance from one or more of at least Rauvolfia caffra Sond., Rhamnus prinoïdes L'Herit. and Toddalia asiatica (L.) Lam.. The outcome of these experimental results is expressions of the impact made by the pertinent plant extracts upon the cells, whereby the degree and the kind of inhibition are disclosed. This in vitro screening method is foreseen to be of importance when an in vivo medical treatment is to be determined. The cells may be the patients' own cells but need not. Active substances discoverable by means of this approach and to be used as a medicament may be present in an aqueous solution or formulated in any adequate manner, which is pharmaceutically acceptable.

The present invention additionally provides a method for producing such a medicament, which could be a composition of compounds. The present invention discloses a method for collecting the plant material to be used for the preparation of such a composition to be used for the treatment of the HIV, AIDS and AIDS related complex in addition to boils and pimples.

The inventors of this invention has to their surprise discovered that the administration of said extract to a human being suffering from AIDS or AIDS related complex implied an in vivo inhibition of a HIV infection in human T lymphocyte cells and mononuclear phagocytic lineage cells infected with HIV. The extract to be administered is a preparation essentially containing at least one or more than one active substance from one or more of at least Rauvolfia caffra Sond., Rhamnus prinoïdes L'Hérit., Toddalia asiatica (L.) Lam.. and Zanthoxylum deremense (Engl.) Kokwaro. The clinical condition of the patients suffering from HIV, AIDS or AIDS related complex becomes remarkable improved following the ingestion of said extract according to a prescribed regimen. This is further substantiated below.

### PHARMACEUTICAL COMPOSITIONS

The compositions used according to this invention are prepared by methods known per se by the skilled art worker.

The compositions used according to this invention may be in the form of powders, solutions, or suspensions, which may or may not be divided in unit dosage form or in the form of capsules or tablets.

The compositions used according to this invention may comprise carriers, diluents, absorption enhancers, tablet disintegrating agents and other ingredients which are conventionally used in the art. Examples of solid carriers are magnesium carbonate, magnesium stearate, dextrin, lactose, sugar, talc, gelatin, pectin, tragacanth, methyl cellulose, sodium carboxymethyl cellulose, low melting waxes and cocoa butter.

The route of administration of the compositions used according to this invention may be any route, which effectively transports the active compound to its site of action.

The regimen for any patient to be treated with the compositions mentioned herein should be determined by those skilled in the art. The daily dose to be administered in therapy can be determined by a physician and will depend on the particular compound employed, on the route of administration and on the age and the condition of the patient.

The present invention is further illustrated by the following examples that, however, are not to be construed as limiting the scope of protection. The features disclosed in the foregoing description and in the following examples may, both separately and in any combination thereof, be material for realising the invention in diverse forms thereof.

### PREFERRED EMBODIMENTS

The following preferred examples of embodiments of the invention are intended to provide a further understanding of the present invention. These examples are illustrative only and are not intended to limit the scope of the invention in any way. Optionally, the compositions disclosed may be formulated as a capsule, which may be a gelatin capsule or the compositions may be formulated as a tablet.

### EXAMPLE 1

This example discloses preferred compositions and formulations hereof for the prophylaxis and treatment of HIV, AIDS and AIDS related complex, which composition comprises each of the following selected dried plant material in the proportions (volume %) as follows:

| **Name of plant** | **Classification** | **Proportion of total volume** |
|---|---|---|
| Rauvolfia caffra Sond. | APOCYNACEAE | 8 % (volume) |
| Rhamnus prinoïdes L'Hérit. | RHAMNACEAE | 7 % (volume) |
| Toddalia asiatica (L.) Lam. | RUTACEAE | 3% (volume) |

In a preferred embodiment of the invention the composition is to be in the form of a powder from which the active substance or active substances are easily extracted at 100 degree Celsius with water, which has been boiling.

To further facilitate the ingestion of this preparation, it might be dissolved in an aqueous mixture containing an aromatic flavour such as a sweetener, e.g. ordinary syrup.

Optionally, the composition may be formulated as a capsule, which may be a gelatin capsule.

Optionally, the composition may be formulated as a tablet.

### EXAMPLE 2

This example discloses preferred compositions, formulations hereof and methods for preparing them for the prophylaxis and/or treatment of HIV, AIDS and AIDS related complex, which composition comprises each of the following selected dried plant material in the proportions (volume %) as shown below.

Preferred procedure is given for the preparation of said composition in an amount to be taken during a 24 hours period with an interval of 8 hours. The treatment period per se is governed by a current clinical examination only.

### (a)

Pulverizing into powder the following amounts of dried plant material consisting essentially of:

| **Name of plant** | **Classification** | **Proportion of total volume** |
|---|---|---|
| Rauvolfia caffra Sond. | APOCYNACEAE | 8% (volume) |
| Rhamnus prinoïdes L'Hérit. | RHAMNACEAE | 7% (volume) |
| Toddalia asiatica (L.) Lam. | RUTACEAE | 3% (volume) |

### (b)

Aqueous extraction by means of hot water, preferably boiling water. The hot water is poured onto the powder of step (a) whereby the powder dissolves.

It is appropriate, that the volume to be ingested is around 150 ml by each administration. An administration schedule for a human adult patient may conveniently comprise administration 3 times daily, each administration appropriately taking place following a meal.

### EXAMPLE 3

This example discloses preferred compositions, formulations hereof and methods for preparing them for the prophylaxis and treatment of HIV, AIDS and AIDS related complex, which composition comprises each of the following selected dried plant material in the proportions (volume %) as shown below.

### (a)

Pulverizing into powder the following amounts (volume %)of dried plant material consisting essentially of:

| **Name of plant** | **Classification** | **Proportion of total volume** |
|---|---|---|
| Rauvolfia caffra Sond. | APOCYNACEAE | 8 % (volume) |
| Rhamnus prinoïdes L'Hérit. | RHAMNACEAE | 7% (volume) |
| Zanthoxylum deremense (Engl.) Kokwaro. | RUTACEAE | 1.5% (volume) |
| Toddalia asiatica (L.) Lam. | RUTACEAE | 1.5 % (volume) |

### (b)

Aqueous extraction by means of hot water, preferably boiling water. The hot water is poured onto the powder of step (a) whereby the powder dissolves.

It is appropriate, that the volume to be ingested is around 150 ml by each administration. An administration schedule for a human adult patient may conveniently comprise administration 3 times daily, each administration appropriately taking place following a meal.

### EXAMPLE 4

This example discloses a preferred composition, formulations hereof into a medicament and methods for preparing it for the prophylaxis and treatment of patients suffering from HIV, AIDS and AIDS related complex. The following dried plant material is selected in the proportions (volume %) as shown below.

| **Name of plant** | **Classification** |
|---|---|
| Rauvolfia caffra Sond. | APOCYNACEAE |
| Rhamnus prinoïdes L'Hérit. | RHAMNACEAE |
| Toddalia asiatica (L.) Lam. | RUTACEAE |

Each plant is collected fresh using the bark of Rauvolfia caffra Sond. and the cortex of the root of Rhamnus prinoïdes L'Hérit. and the cortex of the root of Toddalia asiatica (L.) Lam.. As all of the plants are left alive, the method is considered of good environmental standard. The plant material is roughly cleaned and then thoroughly dried in a well-ventilated shaded area. Each plant component is then separately milled into a fine granulated powder and mixed in accordance with the following formula:

| **Name of plant:** | **Volume of granulated powder** |
|---|---|
| Rauvolfia caffra Sond. | 80 ml (milliliters) |
| Rhamnus prinoïdes L'Hérit. | 70 ml (milliliters) |
| Toddalia asiatica (L.) Lam. | 30 ml (milliliters) |

Thus a total of 180 ml of granulated powder is prepared.

From this mixture 30 ml of granulated powder is filled into an empty thermos container and poured over with 500 ml of boiling water. The thermos is closed, left overnight and thus allowing the hot water to extract the active substance or the active substances from the granulated powder. It is however to be understood that the extracts from each subject plant can be used and prepared by organic extraction procedures known from the prior art.

The dosage for oral use of the fluid extraction chosen for trials is appropriately about 150 ml of this liquid three times a day after meals for an adult human patient.

From trials conducted so far, a therapeutically effective single dose quantity of said composition for an average human adult dose has been found to be at least as follows:

| **Name of plant** | **Classification** | **zVolume of dried plant material** |
|---|---|---|
| Rauvolfia caffra Sond. | APOCYNACEAE | 4.4 ml |
| Rhamnus prinoïdes L'Hérit. | RHAMNACEAE | 3.9 ml |
| Toddalia asiatica (L.) Lam. | RUTACEAE | 1.7 ml |
| Total | | 10.0 ml |

It has been found that the above-mentioned quantities may be varied up to 10-20 % without having adverse clinical significant consequences.

### EXAMPLE 5

In order to consolidate and verify the validity of such an administration to patients in need of a treatment, two adult human patients (patient I and patient II) suffering from HIV, AIDS and AIDS related complex (patient I) and HIV and AIDS (patient II) are medicated according to the present invention and the patients are examined with questionnaire, weight, and blood analyses. Both before the treatment and periodically during the treatment the patients are examined.

Patient I is an adult male and is suffering from HIV, AIDS and AIDS related complex. Patient II is an adult female and is suffering from HIV and AIDS.

The medical examination comprises inter alia the measurement of the below listed standardized clinical-chemistry laboratory parameters. Each parameter is given with approximate values for the normal and pathologic range in addition to the unit of measurement:

Hemoglobin (HB); normal range: 13-18 g/dl (males) and 12-16 g/dl (females). Values below the lower threshold is seen in case of AIDS

White blood cells (WBC); normal range: 4.3-10.8 x 10⁹ / liter.

Viral load (number of copies of HIV RNA per milliliter of serum or plasma): Normal range: Zero. Above zero is pathologic.

CD4+ helper T-lymphocyte cell count (numbers of cells per µl of blood): Normal range: 600-1500 cells/µl. HIV pathologic range: 200-600 cells/µl. AIDS pathologic range: Below 200.

CD8+ killer/suppressor T-lymphocyte cell count (number of cells per cubic millimeter of blood): Normal range: 300-800

CD4+/CD8+ ratio. Normal range: Approximately 2.0 and pathologic range is below 2.0

Body weight. Measured in kg. As a rule of thumb a weight loss of more than 5 % from normal weight would be considered as a warning sign.

To further disclose and clarify the importance of this invention the clinical chemistry parameters measured will be briefly explained hereinafter:

Lymphocytes are an important fraction of the total number of white blood cells.

The CD4+ cells ("helper" T-lymphocytes) and CD8+ cells (killer/suppressor T-lymphocytes) are specialized lymphocytes named after the special proteins called CD4+ and CD8+ that are embedded in the cell surface.
Both cell categories play an active role in the defense against infections in the human body. On the other hand - one of the ways HIV invades cells is by first attaching to the CD4+ molecule (CD4+ receptor) and later destroying the cell.

The CD4+ T lymphocyte cell count is the laboratory test generally accepted as an indicator of the immunologic competence of the patient with HIV infection.

While the CD4+ T lymphocyte cell count give information about the current immunologic status of the patient, the viral load (HIV RNA level) predicts what will happen to that CD4+ T lymphocyte cell count in the near future and thus predicts the clinical prognosis for the patient. A CD4+ count less than 200 qualifies as a diagnosis of AIDS.

The patients included in the pilot study show clinical significant improvement in respect of enhanced appetite, recovering from opportunistic infections, weight gaining and a significant drop in the viral load.

Figures 1-2 show a significant drop in viral load level as well as an improvement in the CD4+ and CD8+ measurements and additionally a rise of hemoglobin has been observed.

### EXAMPLE 6

In a clinical three weeks study patients suffering from boils and pimples during a treatment comprising ingestion of a composition according to Example 2 above were found to have their boils and pimples significantly reduced in number and seriousness or even abolished. Furthermore it was quite surprisingly found that the hair of the treated patients had a stronger and more forceful growth and the (uninfected) skin generally changed character towards that of a healthy person of a younger age.

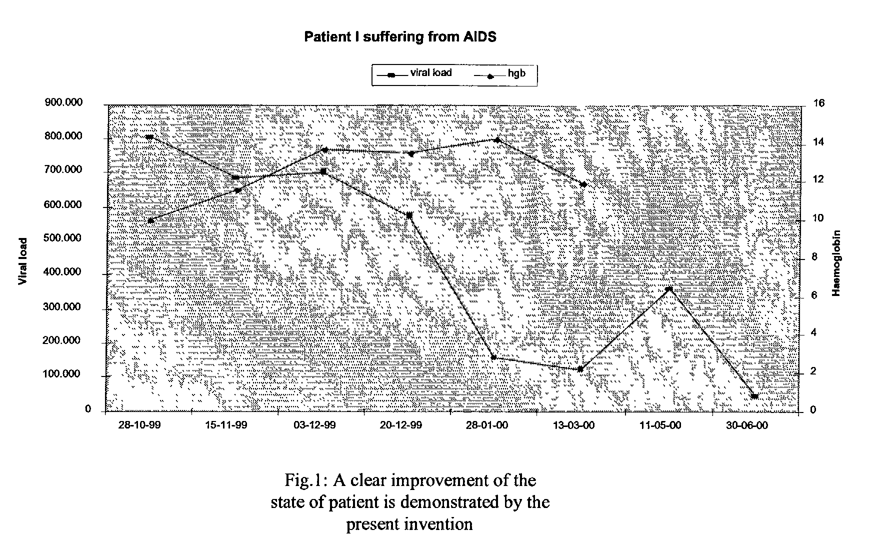

Treatment of an adult male according to the invention improves clearly the pathological state of the patient.

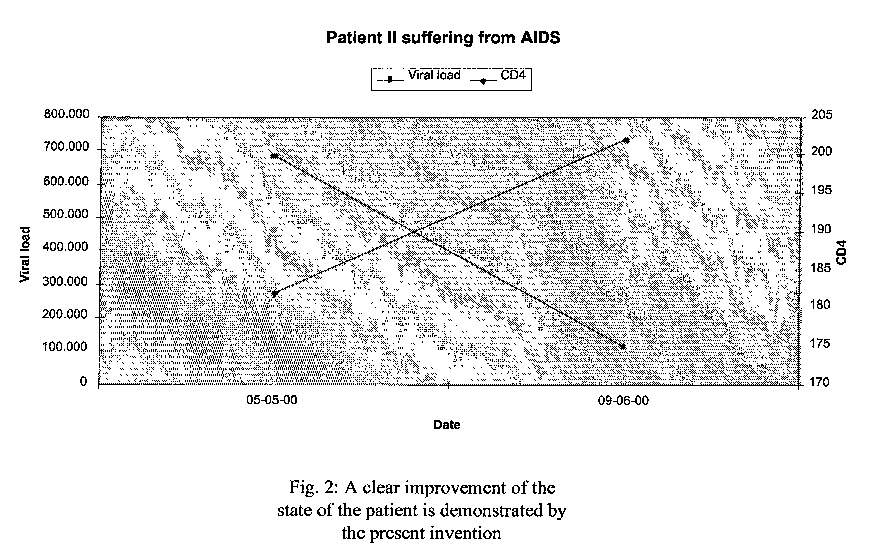

Treatment of an adult female according to the invention improves clearly the pathological state of the patient.

## Claims

1. Composition for the prophylaxis and treatment of infective immunologic disorders, said composition comprising an extract of plant material originating from at least one or more than one active substance of (a) one or more of the plants Rauvolfia caffra Sond., Rhamnus prinoïdes L'Hérit., Toddalia asiatica (L.) Lam. and Zanthoxylum deremense (Engl.) Kokwaro or (b) one or more of Rauvolfia caffra Sond., Rhamnus prinoïdes L'Hérit. and Toddalia asiatica (L.) Lam.

2. Composition according to claim 1, in which the composition is comprising 20-70%, preferably 40-50%, even more preferably 43-47%, of the total volume of the following plants formulated by volume of Rauvolfia caffra Sond., 20-60%, preferably 35-45%, of the total volume formulated by volume of Rhamnus prinoïdes L'Hérit., up till 30%, preferably 5-15%, of the total volume formulated by volume of Toddalia asiatica (L.) Lam., and up till 30%, preferably 5-15%, of the total volume formulated by volume of Zanthoxylum deremense (Engl.) Kokwaro.

3. Composition according to claims 1-2 in which the immunologic disorders are one or more of the disorders belonging to the group of HIV, AIDS, AIDS related complex, boils and pimples.

4. Use of the composition according to claim 3 for production of a pharmaceutical preparation.

5. Use of the pharmaceutical preparation according to claim 4 for production of a medicament.

6. Use of the medicament according to claim 5 for a pharmaceutical preparation for the treatment of infective immunologic disorders.

7. Use of the medicament according to claims 6 in which the immunologic disorders are one or more of the disorders belonging to the group of HIV, AIDS, AIDS related complex, boils and pimples.

8. Method for the prophylaxis and treatment of infective immunologic disorders comprising administering to a patient in need thereof an effective amount of a composition according to claims 1-2.

9. Method according to claim 8 in which the immunologic disorders are one or more of the disorders belonging to the group of HIV, AIDS, AIDS related complex, boils and pimples.

10. Method for in vitro inhibition of HIV infection in human T lymphocyte cells and mononuclear phagocytic lineage cells infected with HIV comprising treatment with an extract of at least one or more of the plants belonging to the group of (a) Rauvolfia caffra Sond., Rhamnus prinoïdes L'Herit., Toddalia (L.) Lam. and Zanthoxylum deremense (Engl.) Kokwaro or (b) one or more of the plants Rauvolfia caffra Sond., Rhamnus prinoïdes L'Hérit. and Toddalia asiatica (L.) Lam.

11. Method for preparation of a pharmaceutical composition comprising an aqueous extract of plant material originating from the group of (a) one or more of Rauvolfia caffra Sond., Rhamnus prinoïdes L'Hérit., Toddalia asiatica (L.) Lam. and Zanthoxylum deremense (Engl.) Kokwaro or (b) one or more of the plants Rauvolfia caffra Sond., Rhamnus prinoïdes L'Hérit. and Toddalia asiatica (L.) Lam.

12. Any novel feature or combination of features described herein.
